# EUROPÄISCHE PATENTSCHRIFT

(11) **EP 2 240 590 B1**
(45) Veröffentlichungstag und Bekanntmachung des Hinweises auf die Patenterteilung: **25.04.2012**
(21) Anmeldenummer: 08860821.1
(22) Anmeldetag: 11.12.2008
(51) Int. Cl.: C12P 5/02, C12N 13/00

(54) **VERFAHREN ZUR ERZEUGUNG VON BIOGAS**
METHOD FOR GENERATING BIOGAS
PROCÉDÉ DE GÉNÉRATION DE BIOGAZ

(30) Priorität: 11.12.2007 DE 102007060687
(43) Veröffentlichungstag der Anmeldung: 20.10.2010
(73) Patentinhaber: Agratec AG, 12489 Berlin (DE)
(72) Erfinder: POLZIN, Manfred, 17166 Gross Wokern (DE)
(74) Vertreter: Reininger, Jan Christian
(86) Internationale Anmeldenummer: PCT/EP2008/067296
(87) Internationale Veröffentlichungsnummer: WO 2009/074635

(56) Entgegenhaltungen:
- EP-A- 1 978 086
- KENNEDY KEVIN J ET AL: "Microwave enhanced digestion of aerobic SBR sludge" WATER SA (PRETORIA), Bd. 33, Nr. 2, April 2007 (2007-04), Seiten 261-270, XP008104970 ISSN: 0378-4738
- ESKICIOGLU C ET AL: "Characterization of soluble organic matter of waste activated sludge before and after thermal pretreatment" WATER RESEARCH, ELSEVIER, AMSTERDAM, NL, Bd. 40, Nr. 20, 1. Dezember 2006 (2006-12-01), Seiten 3725-3736, XP025040017 ISSN: 0043-1354 [gefunden am 2006-12-01]
- YADVIKA ET AL: "Enhancement of biogas production from solid substrates using different techniques - A review" BIORESOURCE TECHNOLOGY OCTOBER 2004 ELSEVIER LTD GB, Bd. 95, Nr. 1, Oktober 2004 (2004-10), Seiten 1-10, XP002522913
- PETERSSON ANNELI ET AL: "Potential bioetanol and biogas production using lignocellulosic biomass from winter rye, oilseed rape and faba bean" BIOMASS AND BIOENERGY, Bd. 31, Nr. 11-12, November 2007 (2007-11), Seiten 812-819, XP022309862 ISSN: 0961-9534

## Beschreibung

Die Erfindung betrifft ein Verfahren zur Erzeugung von Biogas, bei dem organische Substanzen zunächst physikalisch aufgeschlossen werden und anschließend in einem Fermenter mit Hilfe von Mikroorganismen eine anaerobe Vergärung erfolgt.

Bei der anaeroben Vergärung von organischem Material entsteht aufgrund der Stoffwechselaktivität anaerober Mikroorganismen in aufeinander folgenden biochemischen Einzelprozessen ein Gasgemisch aus Methan und Kohlendioxid sowie einer geringen Menge weiterer Gase, das nach einem Reinigungs- und Aufbereitungsprozess zur Stromerzeugung oder als Treibstoff verwendet wird. Ein Teil der bei der Stromerzeugung anfallenden Abwärme wird zur Aufrechterhaltung der in der Biogasanlage für den Fermentationsprozess erforderlichen Temperatur genutzt. Als organische Stoffe werden Bioabfälle, nicht genutzte und/oder zur Biogaserzeugung gezielt angebaute Pflanzen und Pflanzenteile eingesetzt. Eine hervorragend geeignete Konservierungsmethode für als Energieträger von Biogasanlagen verwendete Pflanzen ist die überwiegend in Flach- oder Ballensilos durchgeführte Silage.

Während der biochemischen Prozesse können Kohlehydrate, Fette und Eiweiße in Biogas umgewandelt werden, und zwar zunächst die niedermolekularen Substanzen und unter Einsatz spezieller Enzyme in begrenztem Umfang auch die höhermolekularen Substanzen. Neben der enzymatischen Spaltung schwer abbaubarer Materialen in leichter abbaubare niedermolekulare Stoffe können höhermolekulare Stoffe auch durch chemische oder physikalische Einwirkung oder eine Kombination der bekannten Verfahren aufgeschlossen und dadurch leichter abgebaut werden.

Bei der Verwendung von Ganzpflanzen für die Biogasproduktion ist der Faseranteil, und zwar in Abhängigkeit vom Erntezeitpunkt bzw. dem Reifegrad der Pflanzen, und damit auch der Anteil höhermolekularer Kohlehydrate jedoch besonders hoch. Dieser hohe Anteil höhermolekularer Substanzen kann mit den bekannten Verfahren nur schwer und unvollständig abgebaut werden und steht somit während des Vergärungsprozesses für die Biogaserzeugung nicht zur Verfügung.

"Microwave enhanced digestion of aerobic SBR Sludge" von Kennedy et al., Water SA (Pretoria), Bd. 33, Nr. 2, April 2007 und "Characterization of soluble organic matter..." von Eskicioglu et al., Water Research, Elsevier, Bd. 40, Nr. 20, 1. Dezember 2006 offenbaren jeweils ein Verfahren, bei dem Klärschlamm einer Mikrowellenbestrahlung ausgesetzt wird. Anschließend wird der bestrahlte Klärschlamm einem Biogasherstellungsprozess zugeführt.

Petersson A. et al., Biomass and Bioenergy, Bd. 31, Nr. 11-12, November 2007, Seiten 812-819, offenbart ein Verfahren zur Herstellung von Biogas aus lignocellulosischer Biomasse, bei dem das Ausgangsmaterial durch Nassoxidation vorbehandelt wird.

Der Erfindung liegt die Aufgabe zugrunde, ein Verfahren zur Erzeugung von Biogas anzugeben, mit dem im Biogasprozess bei einem hohen Anteil schwer abbaubarer höhermolekularer Substanzen ein hoher spezifischer Gasertrag erzielt werden kann.

Erfindungsgemäß wird die Aufgabe mit einem Verfahren gemäß den Merkmalen des Patentanspruchs 1 gelöst. Vorteilhafte Weiterbildungen der Erfindung sind Gegenstand der Unteransprüche.

Der Grundgedanke der Erfindung besteht mit anderen Worten darin, dass zur Vergärung in einer Biogasanlage feste, faserreiche Substanzen in Form von Ganzpflanzen oder als Silage konservierten Ganzpflanzen mit einem hohen Anteil an Zelluloseligninen, das heißt, schwer abbaubaren höhermolekularen Stoffen, eingesetzt werden und diese Substanzen vor dem Einbringen in den Fermenter der Biogasanlage mit Mikrowellen von bestimmter Frequenz behandelt werden, bei der die höhermolekularen Bestandteile der Pflanzen in niedermolekulare Substanzen aufgespalten werden und somit dem Vergärungsprozess zugänglich gemacht werden. Auch die nach der Fermentation anfallenden Gärreste, die immer noch höhermolekulare Bestandteile enthalten können, werden mit den Mikrowellen behandelt und anschließend einem erneuten Fermentationsprozess zugeführt. In Abhängigkeit von der Frequenz und Stärke des die Mikrowellen erzeugenden elektromagnetischen Feldes, in dem sich die betreffende Biomasse befindet, verändern die sich nach dem Magnetfeld ausrichtenden mehrpoligen Moleküle der schwer abbaubaren höhermolekularen Bestandteile der Biomasse ständig ihre Lage, so dass Reibungswärme freigesetzt wird und aufgrund der Erwärmung und Ausdehnung des Materials die höhermolekularen Bestandteile aufgespalten werden. Dadurch wird der Anteil der anaerob abbaubaren Bestandteile in der Biomasse und letztlich der Biogasertrag bei der Biogaserzeugung erhöht. Die Mikrowellenvorbehandlung der Biomasse sorgt zudem für das Abtöten schädlicher Bakterien und Pilze, die somit nicht in die Biogasanlage gelangen und mithin dem Vergärungsprozess nicht entgegenwirken.

Die durch Mikrowellenbestrahlung vorbehandelten Stoffe sind vorzugsweise Ganzpflanzen oder als Silage vorliegende Ganzpflanzen mit einem in Abhängigkeit vom Erntezeitpunkt mehr oder weniger hohen Faseranteil und einem dementsprechend hohen Anteil an höhermolekularen, schwerabbaubaren Kohlehydraten.

Die Stärke und Frequenz des die Mikrowellen erzeugenden Magnetfeldes zur Vorbehandlung der Biomasse wird so eingestellt, dass zwar eine Aufspaltung der höhermolekularen Substanzen erfolgt, aber gleichzeitig die durch Erwärmung bedingten Wasserverluste auf ein bestimmtes Maß begrenzt sind.

Gemäß einem weiteren wichtigen Merkmal der Erfindung wird ein Teil der entwässerten und mit Mikrowellen behandelten Gärreste mit zur Vergärung vorgesehener vorbehandelter oder nicht vorbehandelter Biomasse, die dadurch erwärmt und mit Bakterien geimpft wird, vermischt.

Die Mikrowellenbehandlung der Biomasse erfolgt vorzugsweise während der stetigen Zuführung des Biomaterials in den Fermenter, weil die Biomasse während dieses Vorgangs am besten von den Mikrowellen erfasst und durchdrungen wird.

Zum Nachweis des Einflusses einer Mikrowellenbestrahlung auf die Menge des erzeugten Biogases wurde eine aus Ganzpflanzen bestehende Silage, die üblicherweise zur Biogaserzeugung in einen Fermenter einer Biogasanlage eingebracht wird, vor dem Einsatz im Bach-Gärversuch nach VDI 4630 mit Mikrowellen bestrahlt und die dabei erzeugte Gasmenge und Gaszusammensetzung im Vergleich zu dem Gasertrag und der Gaszusammensetzung gemessen, die unter den gleichen Versuchsbedingungen, jedoch ohne Bestrahlung mit Mikrowellen, erzielt wurden. Es wurde festgestellt, dass bei der mit Mikrowellen bestrahlten Silage die Gasmenge und der Methangehalt deutlich höher lagen als bei der Silage ohne Mikrowellenbestrahlung. In Abhängigkeit von der Bestrahlungszeit konnte bei der Vergärung von mit Mikrowellen von 2,45 GHz bestrahlten Ganzpflanzen ein um 30 bis 78% erhöhter Gasgewinn gemessen werden. Eine weitere Untersuchung wurde mit Gärresten durchgeführt, deren flüssiger und fester Anteil getrennt wurde. Der feste Anteil wurde mit Mikrowellen behandelt. Die so aufbereiteten Gärreste, die noch schwer abbaubare, noch nicht in Biogas umgesetzte Kohlehydrate enthalten, wurden in einen in üblicher Weise mit Biomasse beschickten Fermenter für die Biogasproduktion eingebracht. Im Vergleich zur Biogasproduktion in einem Fermenter ohne Zugabe von mit Mikrowellen behandelten festen Gärresten konnte ein Mehrertrag an Gas und Methan gemessen werden. Auch aus separat in einen Fermenter eingebrachten, mit Mikrowellen behandelten Gärresten konnte durch anaerobe Vergärung noch ein zusätzlicher Gasgewinn erzielt werden.

## Patentansprüche

1. Verfahren zur Erzeugung von Biogas, bei dem organische Substanzen zunächst physikalisch aufgeschlossen werden und anschließend in einem Fermenter mit Hilfe von Mikroorganismen eine anaerobe Vergärung erfolgt, **dadurch gekennzeichnet, dass** feste, faserreiche Substanzen in Form von Ganzpflanzen oder als Silage konservierten Ganzpflanzen mit hohem Lignozelluloseanteil vor dem Einbringen in den Fermenter und aus dem Fermenter nach der Vergärung ausgetragene und von Flüssigkeit getrennte Gärreste derart mit Mikrowellen von bestimmter Frequenz behandelt werden, dass unter Begrenzung von Wasserverlusten die höhermolekularen Substanzen der Pflanzen in niedermolekulare Substanzen aufgespalten und darüber hinaus schädliche Bakterien und Pilze abgetötet werden.

2. Verfahren nach Anspruch 1, **dadurch gekennzeichnet, dass** die zur Vergärung vorgesehenen Pflanzen oder konservierten Pflanzen vor dem Einbringen in den Fermenter mit einem Teil der von Flüssigkeit getrennten und mit den Mikrowellen behandelten Gärreste vermischt und dabei erwärmt sowie mit Bakterien beimpft wird, während der restliche Teil der in niedermolekulare Substanzen aufgespaltenen Gärreste einem weiteren Vergärungsprozess zur Erzeugung von Biogas unterzogen wird..

3. Verfahren nach Anspruch 1, **dadurch gekennzeichnet, dass** die organischen Substanzen vor der Fermentation zusätzlich enzymatisch und/oder chemisch und/oder physikalisch aufgeschlossen werden.

## Claims

1. A method for generating biogas, wherein firstly organic substances are disintegrated physically and then an anaerobic fermentation is carried out in a fermenter by means of microorganisms, **characterized in that** solid substances high in fibre and in the form of whole plants or whole plants conserved as silage and having a high lignocellulose fraction prior to insertion into the fermenter and digestates discharged from the fermenter after the fermentation and separated from liquid are treated by microwaves of determined frequency such that by the limitation of water losses the higher-molecular weight substances of the plants are cleaved into low-molecular weight substances and furthermore detrimental bacteria and fungi are killed.

2. The method according to claim 1, **characterized in that** the plants or conserved plants provided for the fermentation are mixed prior to the insertion in the fermenter with a part of the digestates separated from liquid and treated by the microwaves and heated in doing so as well as inoculated by bacteria, whereas the residual part of the digestates cleaved into low-molecular substances is subjected to a further fermentation process for generating biogas.

3. The method according to claim 1, **characterized in that** the organic substances are additionally cleaved enzymatically and/or chemically and/or physically prior to the fermentation.

## Revendications

1. Un procédé de génération de biogaz, dans lequel des substances organiques sont désintégrées physiquement au préalable et ensuite une fermentation anaérobique est effectuée dans un bioréacteur au moyen de microorganismes, **caractérisé en ce que** des substances solides et riches en fibres et sous forme de plantes entières ou plantes entières conservées en ensilage et avec une fraction élevée de lignocellulose avant l'apport dans le bioréacteur et des digestats distribués du bioréacteur après la fermentation et séparés de liquide sont traités par des micro-ondes d'une fréquence déterminée de façon qu'en limitant des pertes d'eau les substances de poids moléculaire élevé des plantes sont désintégrées en substances de faible poids moléculaire et en plus des bactéries et champignons nocifs sont tués.

2. Le procédé selon la revendication 1, **caractérisé en ce que** les plantes ou plantes conservées prévues pour la fermentation sont mélangées avant l'apport dans le bioréacteur avec un part des digestats séparés de liquide et traités par les micro-ondes et échauffées en le faisant ainsi que ensemencées par des bactéries, pendant que le part restant des digestats désintégrés en substances de faible poids moléculaire est soumis à un autre procédé de fermentation de génération de biogaz.

3. Le procédé selon la revendication 1, **caractérisé en ce que** les substances organiques sont désintégrées en outre de façon enzymatique et/ou chimique et/ou physique avant la fermentation.
